# EUROPEAN PATENT SPECIFICATION

(11) **EP 1 496 116 B1**
(45) Date of publication and mention of the grant of the patent: **16.06.2010**
(21) Application number: 03703141.6
(22) Date of filing: 03.02.2003
(51) Int. Cl.: C12N 15/09, C07K 14/245, C07K 19/00, A61K 38/16, A61K 45/00, A61K 47/48, A61K 48/00, A61P 35/00, A61P 43/00, A61K 38/17

(54) **ANTICANCER AGENTS USING VERO TOXIN VARIANTS**
ANTIKREBSMITTEL MIT VEROTOXINVARIANTEN
AGENT ANTICANCEREUX UTILISANT DES VARIANTES DE VEROTOXINES

(30) Priority: 04.02.2002 JP 2002026577
(43) Date of publication of application: 12.01.2005
(73) Proprietor: Yoshida, Hideo, Kyoto-shi, Kyoto 607-8454 (JP)
(72) Inventor: LIU, Xiaoyan, Suita-shi, Osaka 565-0853 (JP)
(74) Representative: Smyth, Gyles Darren
(86) International application number: PCT/JP2003/001043
(87) International publication number: WO 2003/066854

(56) References cited:
- WO-A1-95/22349
- US-A- 5 552 144
- HOVDE C J ET AL: "EVIDENCE THAT GLUTAMIC ACID 167 IS AN ACTIVE-SITE RESIDUE OF SHIGA-LIKE TOXIN I" PROCEEDINGS OF THE NATIONAL ACADEMY OF SCIENCES OF THE UNITED STATES OF AMERICA, vol. 85, no. 8, 1988, pages 2568-2572, XP002333395 ISSN: 0027-8424
- JACKSON M P ET AL: "MUTATIONAL ANALYSIS OF THE SHIGA TOXIN AND SHIGA-LIKE TOXIN II ENZYMATIC SUBUNITS" JOURNAL OF BACTERIOLOGY, WASHINGTON, DC, US, vol. 172, no. 6, June 1990 (1990-06), pages 3346-3350, XP000198709 ISSN: 0021-9193
- GARIEPY JEAN: "The use of Shiga-like toxin 1 in cancer therapy" CRITICAL REVIEWS IN ONCOLOGY-HEMATOLOGY, vol. 39, no. 1-2, July 2001 (2001-07), pages 99-106, XP002333396 ISSN: 1040-8428
- ARAB S ET AL: "VEROTOXIN INDUCES APOPTOSIS AND THE COMPLETE, RAPID, LONG-TERM ELIMINATION OF HUMAN ASTROCYTOMA XENOGRAFTS IN NUDE MICE" ONCOLOGY RESEARCH, PERGAMON PRESS, NEW YORK, NY, US, vol. 11, 1999, pages 33-39, XP000983734 ISSN: 0965-0407
- LINGWOOD C A: "Verotoxin/globotriaosyl ceramide recognition: Angiopathy, angiogenesis and antineoplasia" BIOSCIENCE REPORTS, vol. 19, no. 5, October 1999 (1999-10), pages 345-354, XP002333397 ISSN: 0144-8463

## Description

### TECHNICAL FIELD

The present invention relates to an attenuated verotoxin, its A subunit, an anticancer agent comprising the verotoxin or its A subunit as an active ingredient, and use in the manufacture of a medicament for treating cancer of the anticancer agent. The invention also relates to DNA that encodes the attenuated verotoxin or its A subunit, a vector comprising the DNA, a host cell comprising the vector, a gene therapy agent for treating cancer comprising the vector or host cell as an active ingredient, and use in the manufacture of a medicament for treating cancer of the gene therapy agent. The invention further relates to a complex comprising the attenuated verotoxin or its A subunit with a ligand that specifically and selectively binds to a cancer cell, and an anticancer agent comprising the complex as an active ingredient.

### BACKGROUND ART

Verotoxin (Verotoxin: VT) is a proteinous exotoxin produced by a kind of pathogenic *E. coli,* enterohemorrhagic *E. coli*. This toxin is known to be comprised of one A subunit and five B subunits, the A subunit being toxic and the B subunits having the capability of binding to receptors.

Two types of verotoxin exist: VT1 which is the same as Shiga toxin produced by Shigella dysenteriae type 1, and VT2, which is approximately 60% homologous with VT1.

Currently known members of the verotoxin family include VT1 (Verotoxin 1) (also referred to as "SLT-I" (shiga-like toxin type I)), VT2 (Verotoxin 2)(also termed "SLT-II" (shiga-like toxin type II)), VT2vha (VT2 variant human a), VT2vhb (VT2 variant human b), VT2vp1 (VT2 variant porcine 1) and VT2vp2 (VT2 variant porcine 2).

Verotoxin has N-glycosidase activity towards RNA and hydrolyzes the glycoside bond of the adenosine residue at the 4324^{th} position from the 5'-terminus of the 28S ribosomal RNA, which is a constituent of the 60S subunit of eukaryotic ribosomes, to release adenine. As a result, verotoxin inhibits protein synthesis by inhibiting the elongation factor 1 (EF-1)-dependent binding of aminoacyl-tRNA to ribosomes and causes the cells to die. Thus, verotoxin causes serious diseases such as hemorrhagic colitis, hemolytic uremic syndrome and encephalosis.

Although verotoxin is known to have protein synthesis inhibitory action as mentioned above, not much research on verotoxin has been conducted except for its use as a vaccine.

Therefore, a principal object of the invention is to provide an attenuated verotoxin or its A subunit that is effective for cancer, or a complex of the attenuated verotoxin or A subunit with a ligand etc.

Arab S et al: "Verotoxin Induces Apoptosis and the Complete, Rapid, Long-Term Elimination of Human Astrocytoma Xenografts in Nude Mice" Oncology Research, Pergamon Press, New York, NY, US, vol. 11, 1999, pages 33-39, Gariepy Jean: "The use of Shiga-like toxin 1 in cancer therapy" Critical Reviews in Oncology-Hematology, vol. 39, no. 1-2, July 2001 (2001-07), pages 99-106, and WO95/22349 each disclose use of verotoxins in cancer therapy.

Hovde C J et al.: "Evidence that Glutamic Acid 167 is an Active-Site Residue of Shiga-Like Toxin I" Proceedings of the National Academy of Science of the United States of America, vol. 85, no. 8, 1988, pages 2568-2572 discloses that the verotoxin 1 variant E167D reduces the enzymatic activity and cytotoxicity of verotoxin 1.

Jackson M P et al. "Mutational Analysis of the Shiga Toxin and Shiga-Like Toxin II Enzymatic Subunits" Journal of Bacteriology, Washington, DC, US, vol, 172, no. 6 June 1990 (1990-06), pages 3346-3350 discloses that the verotoxin 1 or 2 variant E167D reduces the enzymatic activity and cytotoxicity of verotoxin 1 or 2.

US5,552,144 discloses that the verotoxin 2 variant E167Q and/or R170Q reduce the enzymatic activity and cytotoxicity of verotoxin 2.

### BRIEF DESCRIPTION OF THE DRAWINGS

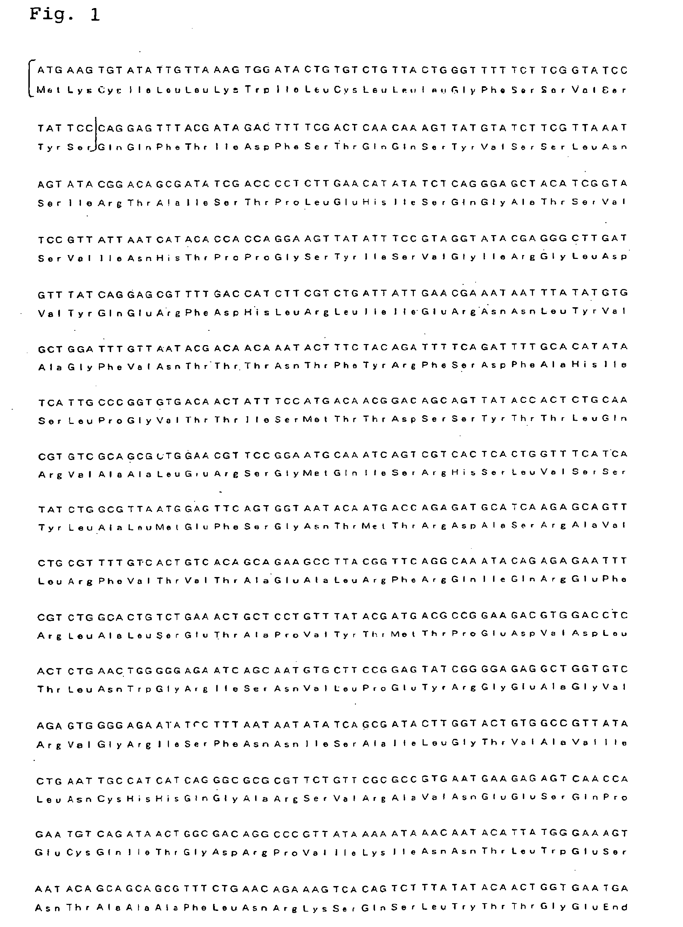

Fig. 1 shows the nucleotide sequence and deduced amino acid sequence of the structural gene of the VT2vp1 A subunit. The 22 amino acid residues from the N terminus and 66 nucleotides from the 5' terminus in the parentheses show the leader sequence.

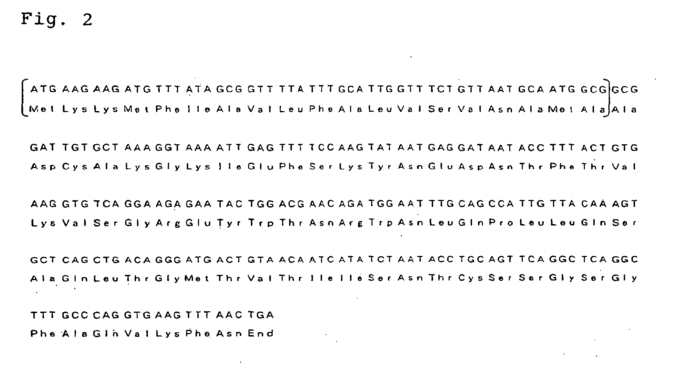

Fig. 2 shows the nucleotide sequence and deduced amino acid sequence of the structural gene of the VT2vp1 B subunit. The 19 amino acid residues from the N terminus and 57 nucleotides from the 5' terminus in the parentheses show the leader sequence.

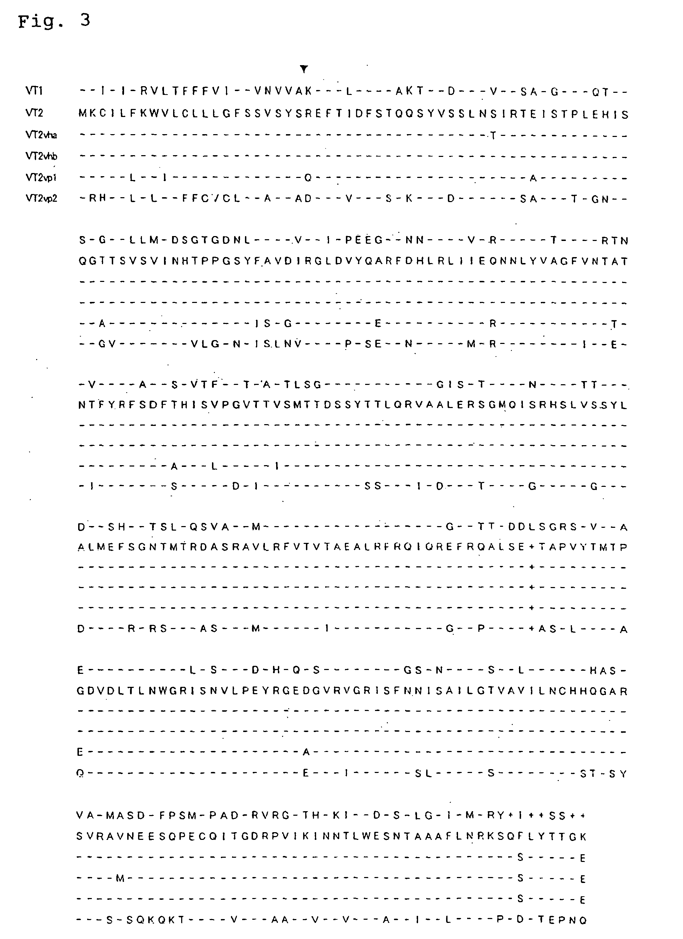

Fig. 3 shows the deduced amino acid sequences of the A subunits of 6 kinds of verotoxins. In Fig. 3, V indicates the position where the processing occurs; - indicates that the amino acid is the same as that of VT2; and + indicates that the amino acid is absent (reference 7).

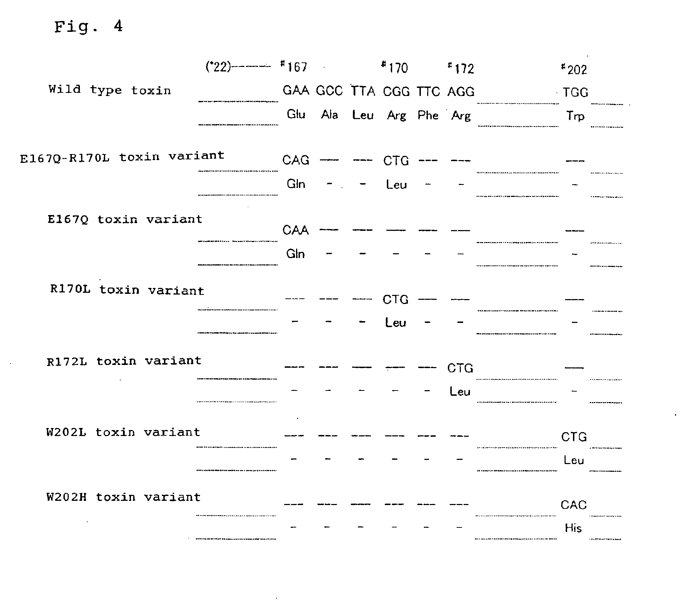

Fig. 4 shows the nucleotide sequences (shown in the upper register) and amino acid sequences (shown in the lower register) of wild type verotoxin and attenuated verotoxins (toxin variants) prepared by a site-directed mutagenesis method. The numbers in Fig. 4 indicate the positions of the amino acid residues of the verotoxin A subunits. # indicates the position of the amino acid residue from the N terminus counted by excluding the 22 leader amino acids; and - indicates that the nucleotide or amino acid residue is the same as that of the wild type verotoxin.

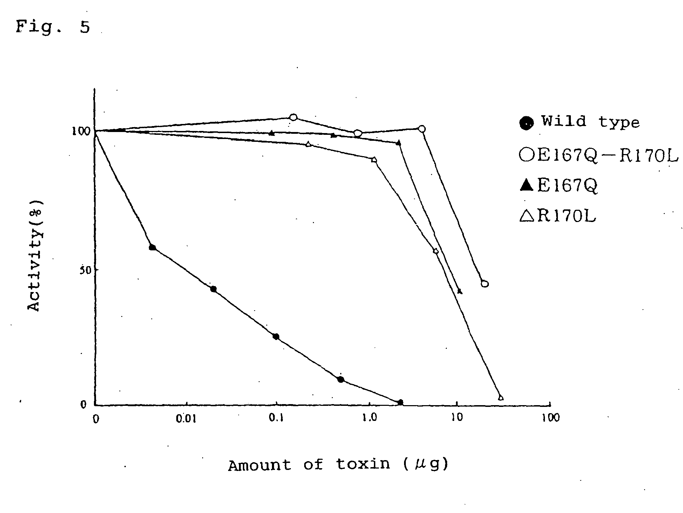

Fig. 5 shows the results of assaying the inhibition of cell-free globin protein synthesis in a rabbit reticulocyte lysate system by a purified wild type VT2vp1 toxin and three kinds of verotoxins of the invention (VT2vpl variants). Protein synthesis activity (%) is plotted on the ordinate and the amount of toxin (µg) is plotted on the abscissa.

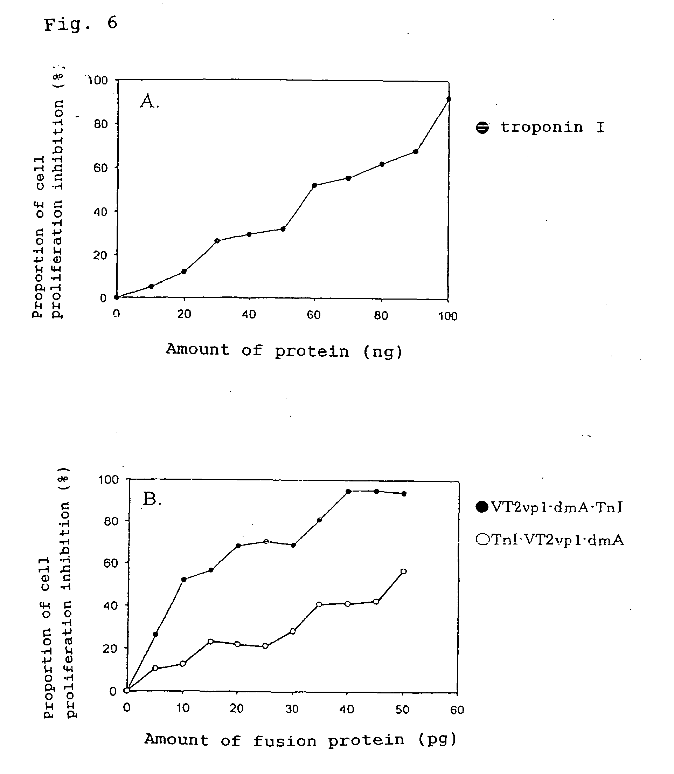

Fig. 6 shows the proportion of cell proliferation inhibition (%) by troponin I protein (Fig. A) and by TnI-VT2vp1-dmA (o) and VT2vp1-dmA-TnI (●) (Fig. B) in fetus umbilical vein endothelial cells. The proportion of endothelial cell proliferation inhibition (%) relative to that of the control is plotted on the ordinate and the amount of protein added per ml of medium is plotted on the abscissa (ng in Fig. A and pg in Fig. B).

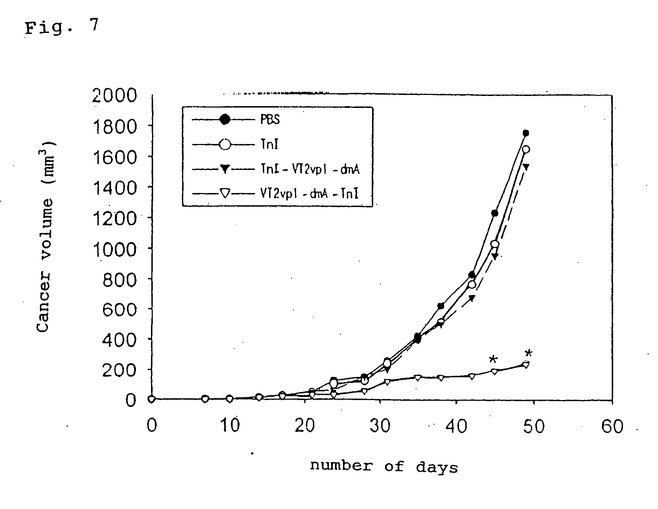

Fig. 7 shows the results of administration of the following substances in the abdominal cavity of female nude mice inoculated with 2 x 10⁶ human ovarian cancer cells (SKOV3): PBS as a control (●); 3 µg of Troponin I protein (o); 3 µg of TnI-VT2vp1-dmA fusion protein (▼); and 3 µg of VT2vp1-dmA-TnI fusion protein (∇). Cancer volume (mm³) is plotted on the ordinate and the number of days after inoculating the cancer cells in the mice is plotted on the abscissa.

### DISCLOSURE OF THE INVENTION

The present inventors found an attenuated verotoxin useful as an anticancer agent. Thus the present invention provides the following:
Item 1. An attenuated verotoxin comprising a verotoxin A subunit amino acid sequence in which the glutamic acid at position 167 from the N terminus is substituted by glutamine and the arginine at position 170 is substituted by leucine.
Item 2. An attenuated verotoxin according to item 1 wherein at least one of the amino acids at positions 168, 169, 171 and 172 and positions 202 to 207 from the N terminus of the verotoxin A subunit is substituted or deleted.
Item 3. An attenuated verotoxin according to item 1 wherein at least one of the amino acids at positions 172 and 202 from the N terminus of the verotoxin A submit is substituted or deleted.
Item 4. An attenuated verotoxin according to item 1 in which the arginine at position 172 of the verotoxin A subunit is substituted by leucine.
Item 5. An attenuated verotoxin according to item 1 in which the tryptophan at position 202 from the N terminus of the verotoxin A subunit is substituted by leucine or histidine.
Item 6. A verotoxin A subunit of the attenuated verotoxin of item 1.
Item 7. DNA that encodes the A subunit of item 6.
Item 8. A vector comprising the DNA of item 7 inserted therein.
Item 9. An anticancer agent comprising the A subunit of the attenuated verotoxin of item 6 or a pharmaceutically acceptable salt thereof as an active ingredient, and a pharmaceutically acceptable carrier, excipient or diluent.
Item 10. An anticancer agent comprising the attenuated verotoxin of item 1 or a pharmaceutically acceptable salt thereof as an active ingredient, and a pharmaceutically acceptable carrier, excipient or diluent.
Item 11. A complex comprising the A subunit of the attenuated verotoxin of item 6 and a ligand that specifically and selectively binds to a cancer cell.
Item 12. A fusion protein comprising the A subunit of the attenuated verotoxin of item 6 and troponin I.
Item 13. A fusion gene comprising the DNA of item 7 and DNA which encodes troponin I.
Item 14. A vector comprising a fusion gene comprising the DNA of item 7 and DNA which encodes troponin I.
Item 15. A complex comprising the attenuated verotoxin of item 1 and a ligand that specifically and selectively binds to a cancer cell.
Item 16. An anticancer agent comprising the complex of item 11 or a pharmaceutically acceptable salt thereof as an active ingredient, and a pharmaceutically acceptable carrier, excipient or diluent.
Item 17. An anticancer agent comprising the fusion protein of item 12 or a pharmaceutically acceptable salt thereof as an active ingredient, and a pharmaceutically acceptable carrier, excipient or diluent.
Item 18. Use of an anticancer agent of any one of items 9, 10, 11, 16 and 17 for the manufacture of a medicament for treating cancer.
Item 19. A gene therapy agent for treating cancer comprising the vector of item 8 or 14 as an active ingredient.
Item 20. Use of the gene therapy agent of item 19 for the manufacture of a medicament for treating cancer.

### DETAILED DESCRIPTION OF THE INVENTION

Verotoxin is a proteinous exotoxin produced by enterohemorrhagic *E. coli* and is composed of an A subunit and B subunits. The kind of *E. coli* from which the verotoxin is derived is not particularly limited. There is also no particular limitation on the kind of toxin, and examples include VT1, VT2, VT2vha, VT2vhb, VT2vp1 and VT2vp2a. VT2vp1 is preferable.

SEQ ID NOs: 1 and 2 and Fig. 1 show the nucleotide sequence and amino acid sequence of the VT2vp1 A subunit, and SEQ ID NOs: 3 and 4 and Fig. 2 show the nucleotide sequence and amino acid sequence of the VT2vp1 B subunit. Fig. 3 shows the amino acid sequences of members of the verotoxin family.

In the A subunit shown in SEQ ID NO: 1 and Fig. 1, the 22 amino acids from the N terminus (parenthesized in Fig. 1) form the leader sequence. Thus, the amino acid sequence numbering used in this specification excludes the leader sequence. That is, an amino acid at position x from the N terminus in this specification refers to the (x+22)^{nd} amino acid from the N terminus in SEQ ID NO: 1. For example, the amino acid at position 10 from the N terminus in this specification means the 32^{nd} amino acid from the N terminus in SEQ ID NO:2.

Similarly, in the subunit B amino acid sequence shown in SEQ ID NO: 4 and Fig. 2, the 19 amino acids from the N terminus (parenthesized in Fig. 2) form the leader sequence.

In the case of DNA sequences, a nucleotide at position y from the 5' terminus of the A subunit DNA sequence in this specification refers to the (y+66)^{th} nucleotide in SEQ ID NO:1. Similarly, a nucleotide at position z from the 5' terminus of the B subunit DNA sequence refers to the (z+57)^{th} nucleotide in SEQ ID NO:3.

### Attenuated verotoxin of the invention

The attenuated verotoxin of the invention (hereinafter sometimes referred to as the "attenuated verotoxin" or "toxin variant") means a verotoxin that minimizes cytotoxicity as much as possible, such as adverse influences on normal cell protein synthesis action, and exhibits an inhibitory action on cancer cell protein synthesis.

The attenuated verotoxin usually may comprise an A subunit and B subunits, but may comprise only the A subunit. The manner of combining the A subunit and B subunits is also not particularly limited. A combination of the subunits of the same verotoxin or different kinds of verotoxins, for example, a combination of the VT2vp1 A subunit and VT2vp2 B subunits can be used.

Based on the fact that the 167th and 170th amino acids of the verotoxin A subunit are active centers of toxin, the present invention provides an attenuated verotoxin by substitution of amino acids at these positions and optionally mutation of amino acids at one or more other positions, so that the resulting attenuated verotoxin can be used as an anticancer agent, an antiviral agent, an antimicrobial agent, etc.

The attenuated verotoxin of the invention comprises a verotoxin A subunit amino acid sequence in which the glutamic acid at position 167 from the N terminus is substituted by glutamine and the arginine at position 170 is substituted by leucine.

In this specification, "mutation" refers to substitution, deletion, insertion or addition. For example, when an amino acid is inserted between positions 172 and 173 in the verotoxin amino acid sequence, an amino acid is inserted at position 172. When an amino acid is inserted at one of the above-mentioned positions, the sequence at positions 200 to 207 is also mutated.

An amino acid can be likewise inserted at positions 200 to 207 in the amino acid sequence.

The number of amino acids to be added is not particularly limited so long as it reduces cytotoxicity and provides anticancer effects. One or more amino acids, preferably one or several amino acids, are added.

In the case of amino acid addition, one or more amino acids may be added at the N terminus or C terminus of the amino acid sequence of the verotoxin A subunit. The number of amino acids to be added is not particularly limited so long as it reduces cytotoxicity and provides anticancer effects. Preferably, one or several amino acids are added.

Preferably, the attenuated verotoxin comprises a verotoxin A subunit amino acid sequence in which one or more of the amino acids at positions 168, 169, 171 and 172 and positions 200 to 207 from the N terminus, preferably one or several amino acids, are substituted or deleted, in addition to the glutamic acid at position 167 from the N terminus being substituted by glutamine and the arginine at position 170 being substituted by leucine.

More preferably, the attenuated verotoxin comprises a verotoxin A subunit amino acid sequence in which at least one of the amino acids at positions 172 and 202 from the N terminus is additionally substituted or deleted. Most preferably, the attenuated verotoxin comprises a verotoxin A subunit amino acid sequence in which at least one of the amino acids at positions 172 and 202 from the N terminus is additionally substituted.

For example, arginine at position 172 is preferably substituted by a neutral amino acid such as glycine, alanine, valine, leucine, isoleucine, phenylalanine, tyrosine, tryptophan, serine, threonine, cystein, methionine, glutamine or asparagine.

Tryptophan at position 202 is preferably substituted by a neutral amino acid as mentioned above or a basic amino acid such as lysine, arginine or histidine.

### Method of producing the attenuated verotoxin of the invention

The method of producing the attenuated verotoxin or its A subunit of the invention (hereinafter sometimes referred to as "protein of the invention") is not particularly limited and known biological or chemical methods may be used.

### (1) Biological methods

### (i) DNA of the invention

Biological methods for obtaining the protein of the invention are not particularly limited and known methods can be used.

For example, DNA that encodes a verotoxin or its A subunit is modified using known means to prepare DNA that encodes the protein of the invention (hereinafter also referred to as "DNA of the invention") and the protein of the invention can be obtained by known genetic engineering techniques using the DNA of the invention.

Means usable for verotoxin DNA modification include, for example, genetic engineering techniques such as site-specific mutagenesis [Methods in Enzymology, 154, 350, 367-382 (1987); ibid, 100, 468 (1983); Nucleic Acids Res., 12, 9441 (1984); Zoku Seikagaku Jikken Koza (Experiments in Biochemistry, Second Series) 1 "Idenshi Kenkyuhouhou (Methods in Gene Research) II" edited by the Japanese Biochemical Society, p105 (1986)]. Such a method can also be carried out using, for example, the site-specific mutation introduction kit "Transformer TM Site-Directed Mutagenesis Kit" of Toyobo Co., Ltd.

In the site-specific mutagenesis method of the invention, the oligonucleotides for introducing nucleotide variations shown in Table 1 are preferably used as primers.

Examples of useful chemical DNA synthesis methods include known chemical synthesis techniques such as the phosphotriester method and the phosphoamidite method [J. Am. Chem. Soc., 89, 4801 (1967); ibid, 91, 3350 (1969); Science, 150, 178 (1968); Tetrahedron Lett., 22, 1859 (1981); ibid, 24, 245 (1983)], and combinations of such techniques.

More specifically, DNA can be synthesized chemically by the phosphoramidite method or the phosphotriester method, or using a commercially available automated oligonucleotide synthesizer.

The DNA of the invention, whose sequence is shown in Fig. 4 together with that of other toxin variants, can be obtained in a manner as mentioned above (Fig. 4 shows the positions where the amino acid sequence of the attenuated verotoxin of the invention and other attenuated verotoxins and the DNA sequence encoding the amino acid sequence are different from those of the wild type).

For example, in the case of DNA that encodes the toxin variant according to the present invention E167Q-R170L (an attenuated verotoxin in which the glutamate at position 167 and arginine at position 170 from the N terminus of verotoxin A subunit are substituted by glutamine and leucine, respectively) G at position 499, A at position 501 and G at position 509 in the verotoxin DNA sequence are changed to C, G and T, respectively. Herein, "A" represents adenine, "T" thymine, "G" guanine, and "C" cytosine.

In the case of DNA that encodes the toxin variant not according to the present invention E167Q (an attenuated verotoxin in which the glutamate at position 167 from the N terminus of the verotoxin A subunit is substituted by glutamine), G at position 499 in the verotoxin DNA sequence is changed to C.

In the case of DNA that encodes the toxin variant not according to the present invention R170L (an attenuated verotoxin in which the arginine at position 170 from the N terminus of the verotoxin A subunit is substituted by leucine), G at position 509 in the verotoxin DNA sequence is changed to T.

In the case of DNA that encodes the toxin variant not according to the present invention R172L (an attenuated verotoxin in which the arginine at position 172 from the N terminus of the verotoxin A subunit is substituted by leucine), A at position 514 and G at position 515 in the verotoxin DNA sequence are changed to C and T, respectively.

In the case of DNA that encodes the toxin variant not according to the present invention W202L (an attenuated verotoxin in which tryptophan at position 202 from the N terminus of the verotoxin A subunit is substituted by leucine), T at position 604 and G at position 605 in the verotoxin DNA sequence are changed to C and T, respectively.

In the case of DNA that encodes the toxin variant not according to the present invention W202H (an attenuated verotoxin in which the tryptophan at position 202 from the N terminus of the verotoxin A subunit is substituted by histidine), "TGG" at positions 604 to 606 in the verotoxin DNA sequence are changed to "CAC".

The DNA of the invention can be preferably amplified by PCR methods [Science, 230, 1350 (1985)]. RACE methods [Rapid amplification of cDNA ends; Jikken Igaku (Experimental Medicine), 12 (6), 35 (1994)], 5'-RACE methods [M. A. Frohman, et al., Proc. Natl. Acad. Sci., USA., 8, 8998 (1988)], etc. can also be used.

The primers used in PCR can be suitably selected with reference to the sequence information on the DNA of the invention and can be synthesized by conventional techniques. The isolation and purification of the amplified DNA can also be carried out by conventional methods, for example by gel electrophoresis.

The nucleotide sequence of the DNA of the invention obtained in the above manner can be determined by conventional techniques, for example, the dideoxy method [Proc. Natl. Acad. Sci., USA., 74, 5463 (1977)] or the Maxam-Gilbert method [Methods in Enzymology, 65, 499 (1980)] or more expediently by means of a commercially available sequencing kit.

### (ii) Protein of the invention

The polypeptide of the invention can be produced by conventional recombinant DNA technology [see, for example, Science, 224, 1431 (1984); Biochem. Biophys. Res. Comm., 130, 692 (1985); Proc. Natl. Acad. Sci., USA., 80, 5990 (1983)], based on the DNA sequence of the invention.

More specifically, the protein of the invention can be produced by a process comprising constructing a recombinant DNA (expression vector) which allows the expression of the DNA of the invention in a host cell, transforming the host cell by introducing the vector, cultivaing the resulting transformant, and harvesting the protein from the culture obtained.

The host cell may be either a prokaryote or eukaryote. Examples of usable prokaryotic hosts are various prokaryotes commonly used, such as *Escherichia coli* and *Bacillus subtilis.* Preferable host cells are those derived from *Escherichia coli,* particularly cells of *E. coli* K12.

Usable eukaryotic host cells are not particularly limited and cells of vertebrates and yeasts, for example, can be used. Examples of preferable vertebrate cells are the monkey cell line COS [Cell, 23: 175 (1981)], Chinese hamster ovary cells and dihydrofolate reductase-defective cell line thereof [Proc. Natl. Acad. Sci., USA., 77: 4216 (1980)] etc. Examples of preferable yeasts are cells of yeasts belonging to the genus *Saccharomyces* etc.

When prokaryotic cells are used as host cells, an expression plasmid prepared by using a vector which is replicatable in the particular host cell and has a promoter and SD (Shine and Dalgarno) sequence upstream of the DNA of the invention, so that the DNA may be expressed therein, as well as an initiation codon (e.g. ATG) necessary for initiation of protein synthesis can be preferably used. As the vector mentioned above, it is usual to employ plasmids derived from *Escherichia coli*, such as pBR322, pBR325, pUC12, pUC13, etc. However, these are not exclusive choices, and various known vectors can be utilized. Examples of commercial vectors for use in expression systems using *E. coli* include pGEX-4T (Amersham Pharmacia Biotech), pMAL-C2, pMA1-P2 (New England Biolabs), pET21, pET21/lacq (Invitrogen) and pBAD/His (Invitrogen).

A vector having a promoter upstream of the DNA of the invention to be expressed, RNA splice site, polyadenylation site and transcription termination sequence is usually used as the expression vector for use when cells of a vertebrate are used as host cells. This vector may further have a replication origin where necessary.

A specific example of the expression vector is pSV2dhfr harboring an early promoter of SV40 [Mol. Cell. Biol., 1: 854 (1981)].

Aside from the above, various known commercially available vectors can be used. Examples of commercial vectors which are used in expression systems using animal cells include vectors for animal cells, such as pEGFP-N, pEGFP-C (Clontech), pIND (Invitrogen), pcDNA3.1/His (Invitrogen), etc., and vectors for insect cells, such as pFastBac HT (GibciBRL), pAcGHLT (PharMingen), pAc5/V5-His, pMT/V5-His and pMT/Bip/V5-his (all Invitrogen).

A specific example of an expression vector for use when yeast cells are used as host cells is pAM82 having a promoter for the acid phosphatase gene [Proc. Natl. Acad. Sci., USA., 80: 1 (1983)]. Examples of commercial expression vectors for yeast cells include pPICZ (invitrogen) and pPICZα (Invitrogen).

Usable promoters are also not particularly limited. When a strain of the genus Escherichia is used as the host, tryptophan (trp) promoters, lpp promoters, lac promoters, recA promoters, PL/PR promoters, etc. can be preferably used. When the host is a strain of the genus *Bacillus, SP01* promoters, *SP02* promoters, penP promoters, etc. are preferably used. When a yeast is used as the host, pH05 promoters, PGK promoters, GAP promoters, ADH promoters, etc. can be preferably used.

Examples of preferable promoters for use when host cells are animal cells include SV40-derived promoters, retrovirus promoters, metallothionein promoters, heat shock promoters, cytomegalovirus promoters, and SRα promoters. Conventional fusion protein expression vectors can also be preferably used as the expression vector for the DNA of the invention. A specific example of such a vector is pGEX (Promega) for the expression of glutathione-S-transferase (GST)-fused proteins.

The polynucleotide sequence which assists in the expression and secretion of a mature polypeptide from host cells, includes, for example, secretory or leader sequences. The protein of the invention can be produced using these sequences or using a marker sequence (hexahistidine tag, histidin tag) used in the purification of a fusion mature polypeptide in the case of bacterial host cells, or a hemagglutinin (HA) tag in the case of mammalian cells.

The method of introducing the desired recombinant DNA (expression vector) into the host cell and the associated transformation method are not particularly limited and various conventional methods can be utilized.

The transformant thus obtained can be cultured in the routine manner, whereby the objective protein of the invention encoded by the appropriately designed gene is expressed and produced (accumulated/secreted) intracellularly, extracellularly or on the cell membrane.

The culture medium to be used can be suitably selected from various commonly used media according to the kind of host cell. The cultivation is also performed under conditions suitable for the growth of the host cell.

The resulting recombinant protein of the invention can be optionally isolated and purified by various separation techniques taking advantage of its physical and/or chemical properties, etc., [see "Seikagaku Data Book (Biochemical Data Book) II", 1175-1259, First Edition, 1st impression, June 23, 1980, Tokyo Kagaku Dojin K.K.; Biochemistry, 25 (25), 8274 (1986); Eur. J. Biochem., 163, 313 (1987), etc.].

Examples of such techniques include conventional reconstitution treatments; treatment with a protein precipitating agent (salting-out method); centrifugation; osmotic shock methods; sonic disruption; ultrafiltration; various types of liquid chromatography such as molecular sieve chromatography (gel filtration), adsorption chromatography, ion exchange chromatography, affinity chromatography, and high performance liquid chromatography (HPLC); dialysis; and combinations of these techniques. A particularly preferable technique is affinity chromatography using a column to which an antibody specific to the protein of the invention has been coupled.

### (2) Chemical methods

The protein of the invention can be produced by standard chemical synthesis methods in accordance with the amino acid sequence shown in Fig. 4. Such methods include conventional liquid-phase and solid-phase methods for peptide synthesis.

More specifically, such peptide synthesis methods include the so-called stepwise elongation method in which the constituent amino acids are coupled one by one for chain extension, based on the amino acid sequence information, and the fragment condensation method which comprises synthesizing fragments each consisting of several amino acids and then coupling the fragments together. The protein of the invention can be synthesized by any of the above methods.

The condensation method for use in the above peptide synthesis may also be a conventional one, including, for example, the azide methods, mixed acid anhydride methods, DCC methods, active ester methods, redox methods, DPPA (diphenylphosphoryl azide) methods, DCC + additive (additive: 1-hydroxybenzotriazole, N-hydroxysuccinamide, N-hydroxy-5-norbornene-2,3-dicarboximide, etc.) methods and Woodward's reagent methods.

The solvent to be used in such methods can also be suitably selected from standard solvents used in peptide condensation reactions. Examples of usable solvents include dimethylformamide (DMF), dimethyl sulfoxide (DMSO), hexaphosphoramide, dioxane, tetrahydrofuran (THF), ethyl acetate, and mixtures thereof.

The carboxyl groups of amino acids or peptides which are not to be involved in the peptide synthesis reaction can be generally protected by esterification, for example in the form of a lower alkyl ester, e.g., methyl ester, ethyl ester, *t-*butyl ester or the like, or an aralkyl ester, e.g., benzyl ester, *p*-methoxybenzyl ester, *p*-nitrobenzyl ester or the like.

The amino acids having a functional group in their side chain, for example, the hydroxyl group of the tyrosine residue, can be protected with an acetyl, benzyl, benzyloxycarbonyl, *t*-butyl or other group, if necessary. The guanidino group of an arginine residue can be protected with a suitable protecting group such as nitro, tosyl, *p*-methoxybenzenesulfonyl, methylene-2-sulfonyl, benzyloxycarbonyl, isobornyloxycarbonyl, adamantyloxycarbonyl or the like.

The deprotection reactions for such protected amino acids, peptides and end product proteins of the invention can also be carried out by known methods, for example, catalytic reduction methods or methods using liquid ammonia/sodium, hydrogen fluoride, hydrogen bromide, hydrogen chloride, trifluoroacetic acid, acetic acid, formic acid, methanesulfonic acid or the like.

The degree of condensation reaction progress at each stage of synthesis can be monitored by known methods such as by ninhydrin reaction. The obtained protein of the invention can be purified by known techniques such as ion exchange chromatography, partition chromatography, gel chromatography, countercurrent distribution and like methods.

### Ligand

The present invention includes a complex comprising the protein of the invention with a ligand that can specifically or selectively bind to a cancer cell.

The ligand is not particularly limited so long as it is capable of specifically or selectively binding to a cancer cell, capillary endothelial cell in cancerous tissue or the like. Biological or synthetic ligands may be used.

Preferably, the ligand has affinity to a substance expressed on the surface layer of cancer cells and specifically or selectively binds to the cell surface substance by electrostatic interaction, hydrophobic interaction, van der Waals interaction, etc.

Examples of ligands specifically or selectively binding to capillary endothelial cells include basic proteins such as Troponin I.

Examples of ligands specifically or selectively binding to cell surface substances include monoclonal antibodies, polyclonal antibodies, antibody fragments containing antigen recognition sites, cell surface receptors, receptor fragments containing ligand binding sites, sugar chains, polypeptides, oligopeptides, amino acids, polynucleotides, oligonucleotides, nucleotides, lipids and the like.

More specifically, examples include anti-integrin antibodies, anti-CD44 antibodies, anti-MUC-1 antibodies, anti-cytokeratin antibodies, anti-epidermal growth factor antibodies, anti-insulin-like growth factor antibodies, anti-insulin-like growth factor receptor antibodies and like antibodies; antibody fragments containing antigen recognition sites; collagen or like polypeptides or oligopeptides; oligopeptides containing an RGD sequence; fibronectin and like glycoproteins; hyaluronic acid, phosphomannan and like polysaccharides; pentamannose-6-phosphate and like oligosaccharides; mannose-6-phosphate and like monosaccharides; and retinoic acid and like vitamin acids.

Due to the lack of interaction with normal cells such as leukocytes and erythrocytes, anti-insulin-like growth factor receptor antibodies, pentamannose-6-phosphate, etc. are preferably used.

Examples of usable synthetic ligands include ligands of synthetic organic compounds and synthetic polymers (or polymer compounds).

The method of binding the ligand to the protein of the invention is not particularly limited and can be suitably selected according to the kind of ligand, etc.

For example, a complex can be formed by mixing the protein of the invention and the ligand, or by known genetic engineering techniques as mentioned above, for example, by constructing a plasmid containing a fusion protein of the attenuated verotoxin A subunit DNA and ligand-encoding DNA. Although the DNA of the invention or ligand-encoding DNA may be positioned at the 5' terminus of the fusion gene, the DNA of the invention is preferably at the 5' end (i.e., the ligand replaces the attenuated verotoxin B subunit).

Examples of particularly preferable embodiments include a fusion protein comprising the attenuated verotoxin A subunit with Troponin I as a ligand. Although the A subunit or troponin I may be positioned at the N terminus, the A subunit is preferably at the N terminus.

### Pharmaceutical composition (anticancer agent)

In the invention, "anticancer agent" refers to pharmaceutical compositions capable of inhibiting the proliferation of cancer cells (tumor cells) or killing cancer cells (tumor cells).

The anticancer agent may contain both the A and B subunits or only the A subunit as an active ingredient. When both the A and B subunits are contained, they may be used concurrently or separately.

The protein or complex of the invention as an active ingredient of the pharmaceutical composition of the invention includes pharmaceutically acceptable salts thereof. Such salts can be prepared by conventional methods in the art. Examples of such salts include alkali metal salts, alkaline-earth metal salts and ammonium salts, such as sodium, potassium, lithium, calcium, magnesium, barium and ammonium salts.

When the active ingredient compound contains a functional group such as an amino group, such salts further includes acid addition salts prepared by reacting the active ingredient of the invention with a suitable organic or inorganic acid. Representative acid addition salts include hydrochlorides, hydrobromides, sulfates, bisulfates, acetates, oxalates, valerates, oleates, laurates, borates, benzoates, lactates, phosphates, *p-*toluenesulfonates (tosylates), citrates, maleates, fumarates, succinates, tartrates, sulfonates, glycolates, ascorbates, benzenesulfonates, naphthalenesulfonates and the like.

The anticancer agent of the invention may comprise a pharmacologically effective amount of the protein or complex of the invention and a suitable carrier, excipient or diluent.

Examples of usable carriers, excipients or diluents include known additives usually used according to the dosage form of the preparation. Examples of such carriers include fillers, volume builders, binders, humectants, disintegrators, surfactants and lubricants.

When the protein of the invention is used as an active ingredient, the pharmaceutical preparation is preferably prepared using various additives which are usually used in protein preparations, such as stabilizers, microbicides, buffers, isotonizing agents, chelating agents, pH regulators and surfactants.

Examples of usable stabilizers include human serum albumin, L-amino acids, saccharides and cellulose derivatives. Such stabilizers may be used singly or in combination with a surfactant or the like. Such a combination may enhance the stability of the active ingredient.

Examples of usable L-amino acids include glycine, cysteine and glutamic acid.

Examples of usable saccharides include monosaccharides such as glucose, mannose, galactose, fructose and the like; sugar alcohols such as mannitol, inositol, xylitol and the like; disaccharides such as sucrose, maltose, lactose and the like; polysaccharides such as dextran, hydroxypropylstarch, chondroitin sulfate, hyaluronic acid and the like; and derivatives thereof.

Examples of usable surfactants include both ionic and nonionic surfactants, such as polyoxyethylene glycol sorbitan alkyl esters, polyoxyethylene alkyl ethers, sorbitan monoacyl esters and fatty acid glycerides.

Preferable examples of cellulose derivatives include methylcellulose, ethylcellulose, hydroxyethylcellulose, hydroxypropylcellulose, hydroxypropylmethylcellulose and carboxymethylcellulose sodium.

The amount of saccharide added is preferably not less than about 0.0001 mg, and more preferably within the range of about 0.01 to about 10 mg, per µg of active ingredient. The amount of surfactant added is preferably not less than about 0.00001 mg, and more preferably within the range of about 0.0001 to about 0.01 mg, per µg of active ingredient. The amount of human serum albumin added is preferably not less than about 0.0001 mg, and more preferably within the range of about 0.001 to about 0.1 mg, per µg of active ingredient. The amount of amino acid added is preferably within the range of about 0.001 to about 10 mg per µg of active ingredient. The amount of cellulose derivative added is preferably not less than about 0.00001 mg, and more preferably within the range of about 0.001 to about 0.1 mg per µg of active ingredient.

The amount of active ingredient in the pharmaceutical preparation of the invention can be suitably selected from a broad range but is usually selected from the range of about 0.00001 to about 70 wt.%, and preferably about 0.0001 to about 5 wt.%.

The pharmaceutical composition may further contain various additives such as buffers, isotonizing agents and chelating agents. Examples of usable buffers include boric acid, phosphoric acid, acetic acid, citric acid, ε-aminocaproic acid, glutamic acid, and/or their corresponding salts (e.g., alkali metal or alkaline earth metal salts, such as sodium salts, potassium salts, calcium salts and magnesium salts). Examples of usable isotonizing agents include sodium chloride, potassium chloride, saccharides and glycerol. Examples of usable chelating agents include sodium edetate and citric acid.

The pharmaceutical preparation of the invention can be provided in the form of a solution, or in a lyophilized form that can be stored. When used, such a lyophilized preparation is dissolved in, for example, water or physical saline or like buffer solutions to achieve a suitable concentration for use.

The unit dosage form of the pharmaceutical preparation of the invention can be selected from various alternatives according to the purpose of treatment. Representative examples thereof include solid dosage forms such as tablets, pills, powders, fine powders, granules, capsules, etc., and liquid dosage forms such as solutions, suspensions, emulsions, syrups and elixirs. These dosage forms can be further classified according to the route of administration as oral, parenteral, nasal, vaginal, rectal (suppository), and sublingual preparations, ointments, etc., and each product can be formulated, molded or otherwise prepared according to standard procedures.

For example, tablets can be prepared using, as the pharmaceutical carrier, excipients such as lactose, sucrose, sodium chloride, glucose, urea, starch, calcium carbonate, kaolin, crystalline cellulose, silicic acid, potassium phosphate and the like; binders such as water, ethanol, propanol, simple syrup, glucose solution, starch solution, gelatin solution, carboxymethylcellulose, hydroxypropylcellulose, methylcellulose, polyvinylpyrrolidone and the like; disintegrators such as carboxymethylcellulose sodium, carboxymethylcellulose calcium, low-substituted hydroxypropylcellulose, dried starch, sodium alginate, agar powder, laminaran powder, sodium hydrogencarbonate, calcium carbonate and the like; surfactants such as polyoxyethylene sorbitan fatty acid esters, sodium laurylsulfate, monoglycerol stearate and the like; disintegration inhibitors such as sucrose, stearin, cacao butter, hydrogenated oil and the like; absorption promoters such as quaternary ammonium bases, sodium laurylsulfate and the like; humectants such as glycerol, starch and the like; adsorbents such as starch, lactose, kaolin, bentonite, colloidal silica and the like; and lubricants such as purified talc, salts of stearic acid, boric acid powder, polyethylene glycol and the like.

Furthermore, tablets may optionally be coated with a standard coating material to provide sugar-coated tablets, gelatin-coated tablets, enteric-coated tablets, film-coated tablets, etc. or even processed into multilayer tablets such as double-layer tablets.

Pills can be prepared using pharmaceutically acceptable carriers such as glucose, lactose, starch, cacao butter, hydrogenated vegetable oil, kaolin, talc and like excipients; powdered gum arabic, powdered tragacanth, gelatin, ethanol and like binders; and laminaran, agar and like disintegrants.

Capsules can be prepared by mixing the active ingredient of the invention with various pharmaceutically acceptable carriers as mentioned above and then filling capsule shells such as hard gelatin capsule shells or soft capsule shells with the resulting mixture according to conventional procedures.

Liquid dosage forms for oral administration include conventional inert diluents such as pharmaceutically acceptable solutions containing water, emulsions, syrups, elixirs and the like, and may further include auxiliary agents such as wetting agents, emulsifiers and suspending agents. These dosage forms can be manufactured according to conventional procedures.

Liquid dosage forms for parenteral administration, such as sterile aqueous or non-aqueous solutions, emulsions and suspensions, can be prepared using diluents such as water, ethyl alcohol, propylene glycol, polyethylene glycol, ethoxylated isostearyl alcohol, polyoxylated isostearyl alcohol, polyoxyethylene sorbitan fatty acid ester and vegetable oils, e.g., olive oil, and may be formulated with an injectable organic ester, such as ethyl oleate. Furthermore, such preparations may be supplemented with conventional solubilizers, buffers, wetting agents, emulsifiers, suspending agents, preservatives, dispersants and like additives.

Sterilization may be carried out by filtration through a bacterial filter, adding a bactericide, irradiation, heating or the like. Furthermore, said preparations can be processed into sterile solid dosage forms which can be dissolved in sterile water or a suitable sterilizable medium immediately before use.

Rectal suppositories or vaginal preparations can be prepared using pharmaceutically acceptable carriers such as polyethylene glycol, cacao butter, higher alcohols, esters of higher alcohols, gelatins, semisynthetic glycerides and the like.

Ointments such as pastes, creams and gels can be manufactured using diluents such as white petrolatum, paraffin, glycerin, cellulose derivatives, propylene glycol, polyethylene glycol, silicone oil, bentonite, and vegetable oils such as olive oil.

Compositions for nasal or sublingual administration can be prepared using well-known standard excipients in the conventional manner.

If necessary, coloring agents, preservatives, aroma chemicals, flavorings, sweeteners, and other medicinal substances can be incorporated in the anticancer agent (pharmaceutical composition) of the invention.

The method of administering the pharmaceutical preparation is not particularly limited and can be suitably selected according to the dosage form, patient variables such as age and sex, severity of illness and other factors. For example, tablets, pills, solutions, suspensions, emulsions, granules and capsules are administered orally. Injections are used alone or mixed with conventional infusion solutions containing a glucose or amino acid, and administered intravenously or, where necessary, administered alone intramuscularly, intradermally, subcutaneously or intraperitoneally. Suppositories are administered rectally; vaginal preparations are administered into the vagina. Nasal preparations are administered through the nostrils; sublingual preparations are administered buccally; and ointments are administered topically for transdermal drug delivery.

The amount of active ingredient in the pharmaceutical preparation and dosage thereof are not particularly limited but can be suitably selected from a broad range according to the desired therapeutic effect, administration method, duration of treatment, patient variables such as age and sex, and other factors. Generally, the daily dosage for adults is usually about 0.1 ng to about 1000 mg, preferably about 0.5 ng to about 100 mg, and particularly preferably about 1 ng to about 100 mg, and can be administered once daily or in several divided doses.

The anticancer agent of the invention is not particularly limited in the type of target cancer to be treated; it can be used for any type of cancer. Examples of target cancers include gastric cancers, lung cancers, esophageal cancers, breast cancers, uterine cancers, liver cancers, pancreatic cancers, colon cancers, cutaneous cancers, laryngeal cancers, prostatic cancers, vesical cancers, kidney cancers, thyroid cancers, brain tumors and malignant lymphomas.

Cancers that cannot be removed by surgery, particularly scattered multiple cancer tissues (e.g., visceral cancers, lymph node metastatic cancers) and cancers recurrent after surgery (e.g., gastric cancers, colon cancers) can be treated by administration of the anticancer agent of the invention by injection using an endoscope. For inoperable cancers (e.g., esophagus cancers, colon cancers), the anticancer agent can be treated by direct injection into the cancerous tissues or by cannula injection into an artery that supplies blood to the cancerous tissues (e.g., liver cancer), etc.

Furthermore, the anticancer agent of the invention can be used alone or in combination with one or more known anticancer agents, hormone drugs, radiation, gene therapy agents of the invention described below, etc.

### Gene therapy agent

The gene therapy agent of the invention is described below in further detail. In practice of the following gene therapy, conventional chemical, molecular biological, microbiological, recombinant DNA, genetic, and immunological techniques can be used. Such techniques are described, for example, in Maniatis, T., et al. (Molecular Cloning: A laboratory manual, Cold Spring Harbor Laboratory, Cold Spring Harbor, New York (1982)), Sambrook, J., et al. (Molecular Cloning: A laboratory manual, 2nd Ed. Cold Spring Harbor Laboratory, Cold Spring Harbor, New York (1981)), Ausbel, F. M., et al. (Current Protocols in Molecular Biology, John Wiley and Sons, New York (1992)), Glover, D. (DNA Cloning, I and II (Oxford Press) (1985)), Anand (Techniques for the Analysis of Complex Genomes (Academic Press) (1992)), Guthrie, G., et al. (Guide to Yeast Genetics and Molecular Biology (Academic Press) (1991)), and Fink, et al. (Hum. Gene Ther., 3, 11-19 (1992)).

The gene therapy agent of the invention is based on the provision of a pharmaceutical composition for inhibition of cancer cell proliferation or cancer cell death by introducing the DNA of the invention into cancer cells or surrounding cells thereof to express the protein of the invention.

In the gene therapy for cancer patients using the DNA of the invention, the desired anticancer (antitumor) effect can be achieved by inserting the DNA of the invention in a retrovirus, adenovirus, or AAV(adeno-associated virus) etc. -derived vector and infecting cancer cells (tumor cells) with the resulting vector to express the protein of the invention.

Vectors which can be used to introduce the DNA of the invention for both recombination and extrachromosomal maintenance are already known in the art and any of such known vectors can be utilized in the invention. For example, a virus vector or plasmid vector which contains a copy of the DNA of the invention linked to an expression control element and which allows the expression of the DNA product in the target cells can be used. Although the gene expression vectors mentioned above can be usually used as such a vector, vectors constructed by using vectors disclosed in US Patent No. 5,252,479 and PCT No. WO 93/07282 (pWP-7A, pWP-19, pWU-1, pWP-8A, pWP-21, pRSVL, etc.) or pRC/CMV (Invitrogen) as source vectors are preferably used. Various viral vectors described hereinafter are particularly preferable.

Promoters specific to the target diseased tissues are preferably used as promoters of the vectors used in gene therapy.

Specific examples thereof are, for the liver, albumin, α-fetoprotein, α1-antitrypsin, transferrin, transthyretin, etc. For the colon, carboxyl anhydrase I, carcinoembrogen antigen, etc. can be used. For the uterus and placenta, estrogen, aromatase cytochrome P450, cholesterol side-chain cleaving P450, 17α-hydroxylase P450, etc. can be used. For the prostate, prostatic antiegn, gp91-fox gene, prostate-specific kallikrein, etc. can be used. For the mamma, erb-B2, erb-B3, β-casein, β-lactoglobin, whey protein, etc. can be used. For the lung, the activator protein C uroglobulin can be used. For the skin, K-14-keratin, human keratin 1 or 6, leucrin, etc. can be used. For the brain, neuroglia fiber acidic protein, mature astrocyte-specific protein, myelin basic protein, tyrosine hydroxylase, etc. can be used. For the pancreas, villin, glucagon, Langerhans' islet amyloid polypeptide, etc. can be used. For the thyroid, thyroglobin, calcitonin, etc. can be used. For bones, α1 collagen, osteocalcin, bone sialoglycoprotein, etc. can be used. For the kidney, renin, liver/bone/kidney alkaline phosphatase, erythropoietin, etc. can be used. For the pancreas, amylase, PAP1, etc. can be used.

The vector for introducing the DNA of the invention can be introduced into cells by various methods known in the art for the introduction of DNA into cells, such as electroporation, calcium phosphate coprecipitation, viral transduction, using gene gun and so forth. The cells transformed with the DNA of the invention can be used per se as a medicine having anticancer effects or as a model system for therapeutic research.

In gene therapy, the vector for introducing the DNA of the invention can be introduced into the target cells of a patient by topical administration to the target tissue site or by systemic administration to the patient by injection. By systemic administration, the subject vector can be delivered to any cells in which mRNA can be expressed. If the transformed DNA cannot be permanently taken up in the chromosomes of the tumor cells, the above administration may be repeated periodically to achieve the desired uptake.

The gene therapy method utilising the invention includes both an in vivo method which comprises administering the material for introducing the DNA of the invention (vector for introducing the DNA of the invention) directly into the body and an ex vivo method which comprises withdrawing the target cells from the patient's body, introducing the gene extracorporeally, and returning the cells into the body. Further, in the method of gene therapy utilising the invention, the gene therapy can be carried by introducing the DNA of the invention directly into cells and then cleaving the RNA chain with the active ribozyme.

More specifically, recombinant virus particles can be prepared by inserting the DNA of the invention into a known recombinant adenovirus gene. Since adenoviruses used in gene therapy are usually designed to lack the early genes E1A and E1B that are essential for replication of the virus from the viral genome, the verotoxin variant A subunit DNA can be inserted into that region.

The recombinant adenovirus containing the DNA of the invention is replicated, for example, in cell line 293 in which the E1A/E1B genes are constantly expressed to provide a large amount of recombinant virus particles. Such virus particles are purified and infected with cancer cells so that the protein of the invention can be produced in cancer cells to inhibit the proliferation of the cancer cells or cause cancer cell death.

The gene therapy agent (composition) of the invention can be prepared by conventional methods using various carriers as mentioned in the pharmaceutical composition section, etc. according to the dosage form.

The gene therapy agent of the invention (pharmaceutical preparation containing the vector for introducing the DNA of the invention) can be prepared in the form in which the vector is entrapped in liposomes or in the form of cultured cells infected with a virus containing a retrovirus vector harboring the DNA of the invention.

These may further be formulated into such forms as in phosphate-buffered physiological saline (pH 7.4), in Ringer's solution, or in an injectable intracellular composition fluid. Furthermore, they may be provided in such forms as can be administered together with a substance capable of enhancing the gene introduction efficiency, such as protamine.

The method of administering the pharmaceutical preparation is not particularly limited and can be suitably selected according to the dosage form, patient variables such as age and sex, severity of illness, and other factors.

The amount of the active ingredient in the pharmaceutical preparation and dosage thereof are not particularly limited and can be suitably selected from a broad range according to the desired therapeutic effect, administration method, duration of treatment, patient variables such as age and sex, and other factors.

Generally, for an adult, the daily dosage of the virus vector containing the DNA of the invention as a pharmaceutical preparation is, for example, about 1×10³ pfu to about 1×10¹⁵ pfu per kilogram body weight, and preferably about 1×10⁵ pfu to about 1×10¹⁰ pfu, in terms of virus titer.

The pharmaceutical preparation can be administered daily at once or several times. The preparation can also be administered intermittently, one to several weeks apart. Preferably, the preparation can be administered in combination with a substance capable of enhancing the gene introduction efficiency, such as protamine, or a pharmaceutical preparation containing such a substance.

The anticancer agent of the invention is not particularly limited in the type of target cancer to be treated; it can be used for any type of cancer. Examples of target cancers include gastric cancers, lung cancers, esophagus cancers, breast cancers, uterine cancers, liver cancers, pancreatic cancers, colon cancers, cutaneous cancers, laryngeal cancers, prostatic cancers, vesical cancers, kidney cancers, thyroid cancers, brain tumors and malignant lymphomas.

Cancers that cannot be removed by surgery, particularly scattered multiple cancer tissues (e.g., visceral cancers, lymph node metastatic cancers) and cancers recurrent after surgery (e.g., gastric cancers, colon cancers) can be treated by administration of the anticancer agent of the invention by injection using an endoscope. For inoperable cancers (e.g., esophagus cancers, colon cancers), the anticancer agent can be treated by direct injection into the cancerous tissues or by cannula injection into an artery that supplies blood to the cancerous tissues (e.g., liver cancer), etc.

Furthermore, the gene therapy agent of the invention can be used alone or in combination with one or more anticancer agents of the invention, known anticancer agents, hormone drugs, radiation, etc.

### References:

(1) Cao, C. et al. Construction of mutant genes for a non-toxic Verotoxin 2 variant (VT2vp1) of Escherichia coli and characterization of purified mutant toxins. Microbiol. Immunol., 38 (6): 441-447, 1994.
(2) Cao, C. et al. Specific detection of a Verotoxin 2 variant, VT2vp1, by a bead-enzyme-linked immunosorbent assay. Microbiol. Immunol., 38 (6): 435-440, 1994.
(3) Noda, M. et al. Purification and some properties of Shiga-like toxin from Escherichia coli O157:H7 that is immunologically identical to Shiga toxin. Microb. Pathog., 2: 339-349, 1987.
(4) Ogazawara, T. et al. Inhibition of protein synthesis by a Vero toxin (VT2 or Shiga-like toxin II) produced by Escherichia coli O157:H7 at the level of elongation factor 1-dependent aminoacyl-tRNA binding to ribosome. Microb. Pathog., 4: 127-135, 1988.
(5) Zhu, L. et al. Sequencing of a cDNA encoding the human fast-twitch skeletal muscle isoform of troponin I. Biochim. Biophys. Acta., 1217: 338-340, 1994.
(6) Moses, M. A. et al. Troponin I is present in human cartilage and inhibits angiogenesis. Proc. Natl. Acad. Sci. USA, 96: 2645-2650, 1999.
(7) Takeda, Y. and Yamazaki, S. Enterohemorragic Escherichia coli and Vero toxins. Clinical and Microorganism, Vol. 18 (4): 5-17, 1991.

### BEST MODE FOR CARRYING OUT THE INVENTION

### Example 1

The oligonucleotides for introducing nucleotide variations shown in the middle column of Table 1 (SEQ ID NOs: 5 to 10, in the order from the above downward) were synthesized using a primer synthesizer (model 391 PCR-Mate) and site-specific mutagenesis was performed using these oligonucleotide primers.

A 1.9-kb Sau3AI fragment containing the VT2vp1 gene was subcloned into M13mp18 and the obtained plasmid (termed "M13mp18.CI") was transformed into *E. coli* CJ236. Then, a single stranded DNA of M13mp18.CI was prepared from the obtained *E. coli*.

The oligonucleotides for introducing nucleotide variants were annealed with the single-stranded DNA and subjected in vitro to a polymerase and ligase chain reaction to provide a double-stranded DNA. The obtained DNA was transformed into *E. coli*. BMH71-81mutS to provide a clone.

A phage was prepared from the *E. coli* clone and infected with *E. coli* MV1184. The double-stranded DNA obtained from the *E*. *coli* was screened to isolate M13mp18.CI containing VT2vp1 variant genes.

Six types of toxin variant genes (DNAs), shown in Fig. 4, were obtained in the above manner. Fig. 4 shows differences of the variants from the wild type in gene sequence (in the upper register) and amino acid sequence (in the lower register), respectively.

These attenuated verotoxin genes and the wild type verotoxin gene respectively were transformed into *E*. *coli* HB101 in such a manner that EcoRI-HindIII fragments containing toxin variants gene were subcloned into pUC118 using the M13mp18.CI.

The obtained *E. coli* HB101 was cultured at 37°C for 48 hours, then ultrasonically treated for 1 minute to disrupt the cells and centrifuged to provide the supernatant as a crude toxin extract. Fig. 4 shows the amino acid sequences of the attenuated verotoxin of the invention and other alternated verotoxins thus obtained (reference 1).

Vero cells were seeded in a 96-well plate at 1 x 10⁴ cells/well and cultured overnight, after which the same volume of each crude toxin extract was added. After 24 hours, the influence of each verotoxin on the Vero cells was confirmed under a microscope. Specific activity was determined in terms of the number of dilutions of toxin variant extract still causing Vero cells to die relative to that of the wild type.

**Table 1**

| Toxin | Oligonucleotide for introducing nucleotide mutations ^{a)} | Specific activity of verotoxin |
|---|---|---|
| Wild type | | 1 |
| Toxin variant E167Q-R170L | 5'-CTGAACAGTAAGGCCTGTGCTG-3' | <1/10,000 |
| Toxin variant E167Q | 5'-CTGAACCGTAAGGCTTGTGCTG-3' | <1/10,000 |
| Toxin variant R170L | 5'-CTGAACAGTAAGGCTTCTGCTG-3' | <1/1,000 |
| Toxin variant R172L | 5'-TGTATTTGCAGGAACCGTAAGG-3' | 1/100 |
| Toxin variant W202L | 5'-CTGATTCTCCCCAGGTTCAG-3' | 1/10 |
| Toxin variant W202H | 5'-GATTCTCCCGTGGTTCAGAGT-3' | 1/10 |

| | | |
|---|---|---|
| a) The underlines indicate positions where variations from the wild type toxin in the nucleotide sequence. | | |

### Example 2

DNAs which encode three types of toxins (E167Q-R170L, E167Q and R170L) having particularly low Vero cell toxicity specific activity, and wild type toxin respectively were inserted into *E. coli* in a manner similar to Example 1. Each *E*. *coli* thus obtained was cultured at 37°C for 48 hours and ultrasonically disrupted. The toxins were then purified by DEAE-Sepharose column chromatography, chromatofocusing chromatography and HPLC (reference 1). The resulting toxin variants were the verotoxin variant of the invention (E167Q-R170L) and two variants not according to the invention (E167Q and R170L) shown in Fig. 4.

These toxin variant proteins were quantitatively determined by Bead-ELISA (reference 2) and subjected to the following experiments.

### Verotoxin test:

Vero cells were cultured in a 96-well plate overnight and the verotoxin variants and wild-type verotoxin at various concentrations prepared by serial dilution were added to the medium. After 48 hours, the cells were observed under a microscope. The mean value of the minimum lethal dose and the maximum nonlethal dose of toxin to cause or not cause the Vero cells to die was defined as CD₅₀ (reference 3). Table 2 shows the results.

### Lethal activity to mice:

DdY mice (Shimizu Laboratory Supply Co., Kyoto) were divided into groups of 5 mice each. The attenuated verotoxin E167Q-R170L) of the invention and two variants (E167Q and R170L) not according to the invention and wild type verotoxin at various concentrations prepared by serial dilution were injected into the abdominal cavity of mice. LD₅₀ of mice was calculated from the number of dead mice and time taken to die (reference 3). Table 2 shows the results.

### Protein synthesis inhibition activity:

Inhibition of cell-free globin synthesis in rabbit reticulocyte lysate was tested. Specific amounts of the attenuated verotoxins and wild type verotoxin were added respectively to 50 µl portions of a reaction mixture containing 15 mM Hepes-KOH buffer (pH 7.6), 15 µM hemin, 100 mM potassium acetate, 1.5 mM magnesium acetate, 0.5 mM spermidine, 2 mM dithiothreitol, 0.2 mM glucose 6-phosphate, 1.5 mM ATP, 0.3 mM GTP, 8mM creatine phosphate, 7.5 µg of creatine kinase, 5 µg of rat liver tRNA, 0.6 µg of globin mRNA, 15 µl of nuclease-treated reticulocyte lysate, 0.1 µl [¹⁴C] leucine, and 30 mM of the other 19 kinds of amino acids, and allowed to react at 37°C for 30 minutes. A portion of each reaction mixture was sampled to determine the radioactivity and the protein synthesis inhibitory activity was thereby calculated (reference 4). Table 2 shows the results.

Fig. 5 shows the influence of amount of various verotoxins of the invention and not according to the invention and wild type toxin on the protein synthesis inhibitory activity.

**Table 2**

| Toxin | Protein synthesis inhibitory activity (ID50^{a)} , µg) | Toxicity to Vero cells (CD50^{b)} , ng) | Lethal activity to mice (LD50^{c)}, µg) |
|---|---|---|---|
| Wild-type | 0.009 | 0.006 | 0.025 |
| Toxin variant E167Q-R170L | 17 | 750 | 50 |
| Toxin variant E167Q | 8.6 | 75 | 12 |
| Toxin variant R170L | 7.1 | 30 | 3 |

| | | | |
|---|---|---|---|
| a) Amount of toxin required to inhibit globin synthesis by 50% b) Amount of toxin required to cause death of 50% of Vero cells c) Amount of toxin required to cause death of 50% of mice Each test group consists of 5 mice. | | | |

### Example 3

Since cancer cell growth is accompanied by vascular cell proliferation, it is considered that inhibition of newborn vascular endothelial cell proliferation can inhibit cancer growth. Therefore, a complex of attenuated verotoxin E167Q-R170L A subunit of the invention with troponin I protein was prepared and the influence of the complex on vascular endothelial cell proliferation was examined.

Troponin I is known to inhibit vascular endothelial cell proliferation and be capable of binding to vascular endothelium. Herein, fusion genes termed TnI-VT2vp1-dmA and VT2vp1-dmA-TnI were prepared by removing the B units from VT2vp1 variant E167-R170L (VT2vp1-dm) gene and ligating human troponin I (TnI) (reference 5) upstream or downstream of the A unit in the following manner.

Gene fragments lacking upstream termination codons were prepared by PCR using a 5'-primer (SEQ ID NO: 11 or 13) of a gene having an EcoRI cutting site inserted at the 5' side and a 3'-primer (SEQ ID NO: 12 or 14) containing a BamHI cutting site in place of the terminal codon.

The gene fragments were digested with EcoRI and BamHI and then inserted between EcoRI and BamHI in the cloning site of pUC119. The resulting plasmids were termed pUC119-VT2vp1-dmA and pUC119-TnI respectively.

The gene fragments lacking downstream initiation codons were prepared by PCR using a 5'-primer (SEQ ID NO: 15 or 17) of a gene having a BamHI cutting site inserted in place of the initiation codon and a 3'-primer (SEQ ID NO: 16 or 18) having a HindIII cutting site inserted at the 3' side.

The gene fragments were digested with BamHI and HindIII and then inserted between BamHI and HindIII in pUC119-VT2vp1-dmA or pUC119-TnI. The resulting plasmids were termed pVT2vp1-dmA-TnI and pTnI-VT2vp1-dmA respectively. The primers used were products of Nippon Flour Mills Co., Ltd.

These fusion genes were then inserted into a plasmid (pTrc99A) and expressed in large amounts in *E. coli* (JM105). The fusion protein products were confirmed by Western blot analysis and purified (reference 6).

Human fetus umbilical vein endothelial cells were inoculated in an amount of 800 cells in a 96-well plate and incubated in DMEM medium containing 5% calf-serum for 3 hours, and then VEGF (vascular endothelial growth factor, 30 ng/ml) was added.

Various concentrations of troponin I protein, VT2vp1-dmA protein (attenuated verotoxin E167 G-R170L protein of the invention), TnI-VT2vp1-dmA protein (troponin I-VT2vp1-dmA fusion protein), and VT2vp1-dmA-TnI protein (VT2vp1-dmA-troponin I fusion protein) were then added.

The proportion of the endothelial cell proliferation inhibition by each protein was calculated from the number of cells after 5 days, relative to the number of control cells in the wells to which only VEGF was added. Fig. 6 shows the results.

Even when VT2vp1-dmA was added to a concentration of 200 ng per ml of medium, no cell proliferation inhibitory effects were observed. To inhibit endothelial cell proliferation by 50%, the required amount of troponin I protein was 60 ng, that of TnI-VT2vp1-dmA fusion protein was 50 pg and that of VT2vp1-dmA-TnI fusion protein was 10 pg. That is, the endothelial cell proliferation inhibitory activities of the fusion proteins TnI-VT2vp1-dmA and VT2vp1-dmA-TnI were 1200 times and 6000 times as high as that of troponin I respectively.

The experimental results demonstrate that the introduction of the attenuated verotoxin A subunits of the invention into proliferating vascular endothelial cells remarkably enhances cell proliferation inhibitory effects.

### Experiment 4

Inhibition of the growth of cancer tissues inoculated into animals was tested.

Human ovary cancer cells (SKOV3) were inoculated into the abdominal cavity of 6- to 8-week-old female nude mice in an amount of 2 x 10⁶ cells. The mice were divided into 4 groups of 4 mice each.

0.8 ml of PBS as a control, 3 µg of troponin I protein, 3 µg of TnI-VT2vp1-dmA fusion protein and 3 µg of VT2vp1-dmA-TnI fusion protein were respectively twice administered into the abdominal cavity of mice, i.e., on day 8 and day 10 when cancer was detectable with the naked eye.

The cancer size was observed for 50 days. The cancers of mice having had troponin I or TnI-VT2vp1-dmA fusion protein injections were slightly smaller compared with those of the control mice having been injected with PBS.

In contrast, the growth of cancer tissues in mice having had VT2vpl-dmA-TnI fusion protein injections was remarkably inhibited. When observed on day 50, the cancer was remarkably smaller compared with that of the control mice having had PBS injections, showing a statistically significant difference from the PBS group (Student's t-test, p< 0.05).

Body weight reduction, reduction in feed consumed or abnormal behavior was not observed in any group.

The above results show that the fusion protein prepared by ligation of troponin I at the C terminal side of the A subunit of the attenuated verotoxin E167 Q-R170L (VT2vp1-dm) of the invention significantly inhibits cancer proliferation in vascular endothelial cells and inhibits cancer tissue growth (Fig. 7).

### INDUSTRIAL APPLICABILITY

Effective non-toxic cancer treatment or inhibition of cancer cell proliferation can be achieved by using the attenuated verotoxin of the invention, its A subunit, or a complex thereof with a ligand.

## Claims

1. An attenuated verotoxin comprising a verotoxin A subunit amino acid sequence in which the glutamic acid at position 167 from the N terminus is substituted by glutamine and the arginine at position 170 is substituted by leucine.

2. A verotoxin A subunit of the attenuated verotoxin of claim 1.

3. DNA that encodes the A subunit of claim 2.

4. A vector comprising the DNA of claim 3 inserted therein.

5. An anticancer agent comprising the A subunit of the attenuated verotoxin of claim 2 or a pharmaceutically acceptable salt thereof as an active ingredient, and a pharmaceutically acceptable carrier, excipient or diluent.

6. An anticancer agent comprising the attenuated verotoxin of claim 1 or a pharmaceutically acceptable salt thereof as an active ingredient, and a pharmaceutically acceptable carrier, excipient or diluent.

7. A complex comprising the A subunit of the attenuated verotoxin of claim 2 and a ligand that specifically and selectively binds to a cancer cell.

8. A fusion protein comprising the A subunit of the attenuated verotoxin of claim 2 and troponin I.

9. A fusion gene comprising the DNA of claim 3 and DNA which encodes troponin I.

10. A vector comprising a fusion gene comprising the DNA of claim 3 and DNA which encodes troponin I.

11. A complex comprising the attenuated verotoxin of claim 1 and a ligand that specifically and selectively binds to a cancer cell.

12. An anticancer agent comprising the complex of claim 7 or a pharmaceutically acceptable salt thereof as an active ingredient, and a pharmaceutically acceptable carrier, excipient or diluent.

13. An anticancer agent comprising the fusion protein of claim 8 or a pharmaceutically acceptable salt thereof as an active ingredient, and a pharmaceutically acceptable carrier, excipient or diluent.

14. The use of an anti cancer agent according to any one of Claims 5, 6, 12 and 13, in the manufacture of a medicament for treating cancer.

15. A gene therapy agent for treating cancer comprising the vector of claim 4 or 10 as an active ingredient.

16. The use of a gene therapy agent according to Claim 15 in the manufacture of a medicament for the treatment of cancer.

## Patentansprüche

1. Abgeschwächtes Verotoxin, umfassend eine Aminosäuresequenz einer Verotoxin A-Untereinheit, worin die Glutaminsäure an Position 167 vom N-Terminus durch Glutamin substituiert ist und das Arginin an Position 170 durch Leucin substituiert ist.

2. Verotoxin A-Untereinheit des abgeschwächten Verotoxins nach Anspruch 1.

3. DNA, die für die A-Untereinheit nach Anspruch 2 kodiert.

4. Vektor, umfassend die DNA nach Anspruch 3, der darin insertiert ist.

5. Antikrebsmittel, umfassend die A-Untereinheit des abgeschwächten Verotoxins nach Anspruch 2 oder ein pharmazeutisch verträgliches Salz davon als einen Wirkstoff und einen pharmazeutisch verträglichen Träger, einen pharmazeutisch verträglichen Hilfsstoff oder ein pharmazeutisch verträgliches Verdünnungsmittel.

6. Antikrebsmittel, umfassend das abgeschwächte Verotoxin nach Anspruch 1 oder ein pharmazeutisch verträgliches Salz davon als einen Wirkstoff und einen pharmazeutisch verträglichen Träger, einen pharmazeutisch verträglichen Hilfsstoff oder ein pharmazeutisch verträgliches Verdünnungsmittel.

7. Komplex, umfassend die A-Untereinheit des abgeschwächten Verotoxins nach Anspruch 2 und einen Liganden, der spezifisch und selektiv an eine Krebszelle bindet.

8. Fusionsprotein, umfassend die A-Untereinheit des abgeschwächten Verotoxins nach Anspruch 2 und Troponin I.

9. Fusionsgen, umfassend die DNA nach Anspruch 3 und DNA, die für Troponin I kodiert.

10. Vektor, umfassend ein Fusionsgen, das die DNA nach Anspruch 3 umfasst und DNA, die für Troponin I kodiert.

11. Komplex, umfassend das abgeschwächte Verotoxin nach Anspruch 1 und einen Liganden, der spezifisch und selektiv an eine Krebszelle bindet.

12. Antikrebsmittel, umfassend den Komplex nach Anspruch 7 oder ein pharmazeutisch verträgliches Salz davon als einen Wirkstoff und einen pharmazeutisch verträglichen Träger, einen pharmazeutisch verträglichen Hilfsstoff oder ein pharmazeutisch verträgliches Verdünnungsmittel.

13. Antikrebsmittel, umfassend das Fusionsprotein nach Anspruch 8 oder ein pharmazeutisch verträgliches Salz davon als einen Wirkstoff und einen pharmazeutisch verträglichen Träger, einen pharmazeutisch verträglichen Hilfsstoff oder ein pharmazeutisch verträgliches Verdünnungsmittel.

14. Verwendung eines Antikrebsmittels nach einem der Ansprüche 5, 6, 12 und 13 bei der Herstellung eines Arzneimittels zur Behandlung von Krebs.

15. Gentherapie-Mittel zur Behandlung von Krebs, umfassend den Vektor nach Anspruch 4 oder 10 als einen Wirkstoff.

16. Verwendung eines Gentherapie-Mittels nach Anspruch 15 bei der Herstellung eines Arzneimittels zur Behandlung von Krebs.

## Revendications

1. Verotoxine atténuée comprenant une séquence d'acides aminés de la sous-unité A de verotoxine dans laquelle l'acide glutamique à la position 167 de la terminaison N est substitué par la glutamine et l'arginine à la position 170 est substituée par la leucine.

2. Sous-unité A de verotoxine de la verotoxine atténuée de la revendication 1.

3. ADN qui code la sous-unité A de la revendication 2.

4. Vecteur comprenant l'ADN de la revendication 3 inséré dedans.

5. Agent anticancéreux comprenant la sous-unité A de la verotoxine atténuée de la revendication 2 ou un sel pharmaceutiquement acceptable de la même en tant que principe actif et un véhicule, un excipient ou un diluant pharmaceutiquement acceptable.

6. Agent anticancéreux comprenant la verotoxine atténuée de la revendication 1 ou un sel pharmaceutiquement acceptable de la même en tant que principe actif et un véhicule, un excipient ou un diluant pharmaceutiquement acceptable.

7. Complexe comprenant la sous-unité A de la verotoxine atténuée de la revendication 2 et un ligand qui se lie spécifiquement et sélectivement à une cellule cancéreuse.

8. Protéine de fusion comprenant la sous-unité A de la verotoxine atténuée de la revendication 2 et la troponine I.

9. Gène de fusion comprenant l'ADN de la revendication 3 et un ADN qui code la troponine I.

10. Vecteur comprenant un gène de fusion comprenant l'ADN de la revendication 3 et un ADN qui code la troponine I.

11. Complexe comprenant la verotoxine atténuée de la revendication 1 et un ligand qui se lie spécifiquement et sélectivement à une cellule cancéreuse.

12. Agent anticancéreux comprenant le complexe de la revendication 7 ou un sel pharmaceutiquement acceptable du même en tant que principe actif et un véhicule, un excipient ou un diluant pharmaceutiquement acceptable.

13. Agent anticancéreux comprenant la protéine de fusion de la revendication 8 ou un sel pharmaceutiquement acceptable de la même en tant que principe actif et un véhicule, un excipient ou un diluant pharmaceutiquement acceptable.

14. L'utilisation d'un agent anticancéreux selon l'une quelconque des revendications 5, 6, 12 et 13, dans la fabrication d'un médicament pour le traitement du cancer.

15. Agent de thérapie génique pour le traitement du cancer comprenant le vecteur de la revendication 4 ou 10 en tant que principe actif.

16. L'utilisation d'un agent de thérapie génique selon la revendication 15 dans la fabrication d'un médicament pour le traitement du cancer.


### SEQUENCE LISTING

<110> YOSHIDA, Hideo
<120> Anticancer agents using verotoxin variants
<130> EPP90392
<140> WO03066854
   <141> 2002-02-03
<150> JP2002-26577
   <151> 2002-02-04
<160> 18
<170> Patent In Ver. 2.1
<210> 1
   <211> 960
   <212> DNA
   <213> Verotoxin 2 variant porcin 1 A subunit
<220>
   <221> CDS
   <222> (1)..(960)
<400> 1
   atg aag tgt ata ttg tta aag tgg ata ctg tgt ctg tta ctg ggt ttt      48
   Met Lys Cys Ile Leu Leu Lys Trp Ile Leu Cys Leu Leu Leu Gly Phe
    1               5                  10                  15
   tct tcg gta tcc tat tcc cag gag ttt acg ata gac ttt tcg act caa      96
   Ser Ser Val Ser Tyr Ser Gln Glu Phe Thr Ile Asp Phe Ser Thr Gln
               20                  25                  30
   caa agt tat gta tct tcg tta aat agt ata cgg aca gcg ata tcg acc     144
   Gln Ser Tyr Val Ser Ser Leu Asn Ser Ile Arg Thr Ala Ile Ser Thr
           35                  40                  45
   cct ctt gaa cat ata tct cag gga gct aca tcg gta tcc gtt att aat     192
   Pro Leu Glu His Ile Ser Gln Gly Ala Thr Ser Val Ser Val Ile Asn
       50                  55                  60
   cat aca cca cca gga agt tat att tcc gta ggt ata cga ggg ctt gat     240
   His Thr Pro Pro Gly Ser Tyr Ile Ser Val Gly Ile Arg Gly Leu Asp
   65                  70                  75                  80
   gtt tat cag gag cgt ttt gac cat ctt cgt ctg att att gaa cga aat     288
   Val Tyr Gln Glu Arg Phe Asp His Leu Arg Leu Ile Ile Glu Arg Asn
                   85                  90                  95
   aat tta tat gtg gct gga ttt gtt aat acg aca aca aat act ttc tac     336
   Asn Leu Tyr Val Ala Gly Phe Val Asn Thr Thr Thr Asn Thr Phe Tyr
               100                 105                 110
   aga ttt tca gat ttt gca cat ata tca ttg ccc ggt gtg aca act att     384
   Arg Phe Ser Asp Phe Ala His Ile Ser Leu Pro Gly Val Thr Thr Ile
           115                 120                 125
   tcc atg aca acg gac agc agt tat acc act ctg caa cgt gtc gca gcg     432
   Ser Met Thr Thr Asp Ser Ser Tyr Thr Thr Leu Gln Arg Val Ala Ala
       130                 135                 140
   ctg gaa cgt tcc gga atg caa atc agt cgt cac tca ctg gtt tca tca     480
   Leu Glu Arg Ser Gly Met Gln Ile Ser Arg His Ser Leu Val Ser Ser
   145                 150                 155                 160
   tat ctg gcg tta atg gag ttc agt ggt aat aca atg acc aga gat gca     528
   Tyr Leu Ala Leu Met Glu Phe Ser Gly Asn Thr Met Thr Arg Asp Ala
                   165                 170                 175
   tca aga gca gtt ctg cgt ttt gtc act gtc aca gca gaa gcc tta cgg     576
   Ser Arg Ala Val Leu Arg Phe Val Thr Val Thr Ala Glu Ala Leu Arg
               180                 185                 190
   ttc agg caa ata cag aga gaa ttt cgt ctg gca ctg tct gaa act gct     624
   Phe Arg Gln Ile Gln Arg Glu Phe Arg Leu Ala Leu Ser Glu Thr Ala
           195                 200                 205
   cct gtt tat acg atg acg ccg gaa gac gtg gac ctc act ctg aac tgg     672
   Pro Val Tyr Thr Met Thr Pro Glu Asp Val Asp Leu Thr Leu Asn Trp
       210                 215                 220
   ggg aga atc agc aat gtg ctt ccg gag tat cgg gga gag gct ggt gtc     720
   Gly Arg Ile Ser Asn Val Leu Pro Glu Tyr Arg Gly Glu Ala Gly Val
   225                 230                 235                 240
   aga gtg ggg aga ata tcc ttt aat aat ata tca gcg ata ctt ggt act     768
   Arg Val Gly Arg Ile Ser Phe Asn Asn Ile Ser Ala Ile Leu Gly Thr
                   245                 250                 255
   gtg gcc gtt ata ctg aat tgc cat cat cag ggc gcg cgt tct gtt cgc     816
   Val Ala Val Ile Leu Asn Cys His His Gln Gly Ala Arg Ser Val Arg
               260                 265                 270
   gcc gtg aat gaa gag agt caa cca gaa tgt cag ata act ggc gac agg     864
   Ala Val Asn Glu Glu Ser Gln Pro Glu Cys Gln Ile Thr Gly Asp Arg
           275                 280                 285
   ccc gtt ata aaa ata aac aat aca tta tgg gaa agt aat aca gca gca     912
   Pro Val Ile Lys Ile Asn Asn Thr Leu Trp Glu Ser Asn Thr Ala Ala
       290                 295                 300
   gcg ttt ctg aac aga aag tca cag tct tta tat aca act ggt gaa tga     960
   Ala Phe Leu Asn Arg Lys Ser Gln Ser Leu Tyr Thr Thr Gly Glu
   305                 310                 315                 320
<210> 2
   <211> 319
   <212> PRT
   <213> Verotoxin 2 variant porcin 1 A subunit
<400> 2
   Met Lys Cys Ile Leu Leu Lys Trp Ile Leu Cys Leu Leu Leu Gly Phe
   1               5                   10                  15
   Ser Ser Val Ser Tyr Ser Gln Glu Phe Thr Ile Asp Phe Ser Thr Gln
               20                  25                  30
   Gln Ser Tyr Val Ser Ser Leu Asn Ser Ile Arg Thr Ala Ile Ser Thr
           35                  40                  45
   Pro Leu Glu His Ile Ser Gln Gly Ala Thr Ser Val Ser Val Ile Asn
       50                  55                  60
   His Thr Pro Pro Gly Ser Tyr Ile Ser Val Gly Ile Arg Gly Leu Asp
   65                  70                  75                  80
   Val Tyr Gln Glu Arg Phe Asp His Leu Arg Leu Ile Ile Glu Arg Asn
                   85                  90                  95
   Asn Leu Tyr Val Ala Gly Phe Val Asn Thr Thr Thr Asn Thr Phe Tyr
               100                 105                 110
   Arg Phe Ser Asp Phe Ala His Ile Ser Leu Pro Gly Val Thr Thr Ile
           115                 120                 125
   Ser Met Thr Thr Asp Ser Ser Tyr Thr Thr Leu Gln Arg Val Ala Ala
       130                 135                 140
   Leu Glu Arg Ser Gly Met Gln Ile Ser Arg His Ser Leu Val Ser Ser
   145                 150                 155                 160
   Tyr Leu Ala Leu Met Glu Phe Ser Gly Asn Thr Met Thr Arg Asp Ala
                   165                 170                 175
   Ser Arg Ala Val Leu Arg Phe Val Thr Val Thr Ala Glu Ala Leu Arg
               180                 185                 190
   Phe Arg Gln Ile Gln Arg Glu Phe Arg Leu Ala Leu Ser Glu Thr Ala
           195                 200                 205
   Pro Val Tyr Thr Met Thr Pro Glu Asp Val Asp Leu Thr Leu Asn Trp
       210                 215                 220
   Gly Arg Ile Ser Asn Val Leu Pro Glu Tyr Arg Gly Glu Ala Gly Val
   225                 230                 235                 240
   Arg Val Gly Arg Ile Ser Phe Asn Asn Ile Ser Ala Ile Leu Gly Thr
                   245                 250                 255
   Val Ala Val Ile Leu Asn Cys His His Gln Gly Ala Arg Ser Val Arg
               260                 265                 270
   Ala Val Asn Glu Glu Ser Gln Pro Glu Cys Gln Ile Thr Gly Asp Arg
           275                 280                 285
   Pro Val Ile Lys Ile Asn Asn Thr Leu Trp Glu Ser Asn Thr Ala Ala
       290                 295                 300
   Ala Phe Leu Asn Arg Lys Ser Gln Ser Leu Tyr Thr Thr Gly Glu
   305                 310                 315
<210> 3
   <211> 264
   <212> DNA
   <213> Verotoxin 2 variant porcin 1 B subunit
<220>
   <221> CDS
   <222> (1)..(264)
<400> 3
   atg aag aag atg ttt ata gcg gtt tta ttt gca ttg gtt tct gtt aat    48
   Met Lys Lys Met Phe Ile Ala Val Leu Phe Ala Leu Val Ser Val Asn
    1               5                  10                  15
   gca atg gcg gcg gat tgt gct aaa ggt aaa att gag ttt tcc aag tat    96
   Ala Met Ala Ala Asp Cys Ala Lys Gly Lys Ile Glu Phe Ser Lys Tyr
               20                  25                  30
   aat gag gat aat acc ttt act gtg aag gtg tca gga aga gaa tac tgg   144
   Asn Glu Asp Asn Thr Phe Thr Val Lys Val Ser Gly Arg Glu Tyr Trp
           35                  40                  45
   acg aac aga tgg aat ttg cag cca ttg tta caa agt gct cag ctg aca   192
   Thr Asn Arg Trp Asn Leu Gln Pro Leu Leu Gln Ser Ala Gln Leu Thr
       50                  55                  60
   ggg atg act gta aca atc ata tct aat acc tgc agt tca ggc tca ggc   240
   Gly Met Thr Val Thr Ile Ile Ser Asn Thr Cys Ser Ser Gly Ser Gly
   65                  70                  75                  80
   ttt gcc cag gtg aag ttt aac tga                                   264
   Phe Ala Gln Val Lys Phe Asn
                   85
<210> 4
   <211> 87
   <212> PRT
   <213> Verotoxin 2 variant porcin 1 B subunit
<400> 4
   Met Lys Lys Met Phe Ile Ala Val Leu Phe Ala Leu Val Ser Val Asn
    1               5                  10                  15
   Ala Met Ala Ala Asp Cys Ala Lys Gly Lys Ile Glu Phe Ser Lys Tyr
               20                  25                  30
   Asn Glu Asp Asn Thr Phe Thr Val Lys Val Ser Gly Arg Glu Tyr Trp
           35                  40                  45
   Thr Asn Arg Trp Asn Leu Gln Pro Leu Leu Gln Ser Ala Gln Leu Thr
       50                  55                  60
   Gly Met Thr Val Thr Ile Ile Ser Asn Thr Cys Ser Ser Gly Ser Gly
   65                  70                  75                  80
   Phe Ala Gln Val Lys Phe Asn
<210> 5
   <211> 22
   <212> DNA
   <213> Artificial Sequence
<220>
   <223> Description of Artificial Sequence: synthetic oligomer
<400> 5
   ctgaacagta aggcctgtgc tg 22
<210> 6
   <211> 22
   <212> DNA
   <213> Artificial Sequence
<220>
   <223> Description of Artificial Sequence: synthetic oligomer
<400> 6
   ctgaaccgta aggcttgtgc tg 22
<210> 7
   <211> 22
   <212> DNA
   <213> Artificial Sequence
<220>
   <223> Description of Artificial Sequence: synthetic oligomer
<400> 7
   ctgaacagta aggcttctgc tg 22
<210> 8
   <211> 22
   <212> DNA
   <213> Artificial Sequence
<220>
   <223> Description of Artificial Sequence: synthetic oligomer
<400> 8
   tgtatttgca ggaaccgtaa gg 22
<210> 9
   <211> 20
   <212> DNA
   <213> Artificial Sequence
<220>
   <223> Description of Artificial Sequence: synthetic oligomer
<400> 9
   ctgattctcc ccaggttcag 20
<210> 10
   <211> 21
   <212> DNA
   <213> Artificial Sequence
<220>
   <223> Description of Artificial Sequence: synthetic oligomer
<400> 10
   gattctcccg tggttcagag t 21
<210> 11
   <211> 37
   <212> DNA
   <213> Artificial Sequence
<220>
   <223> Description of Artificial Sequence: 5'-primer
<400> 11
   ttttgaattc atgaagtgta tattgttaaa gtggata 37
<210> 12
   <211> 37
   <212> DNA
   <213> Artificial Sequence
<220>
   <223> Description of Artificial Sequence: 3'-primer
<400> 12
   ttttggatcc ttcaccagtt gtatataaag actgtga 37
<210> 13
   <211> 37
   <212> DNA
   <213> Artificial Sequence
<220>
   <223> Description of Artificial Sequence: 5'-primer
<400> 13
   ttttgaattc atgggagatg aggagaagcg gaacagg 37
<210> 14
   <211> 37
   <212> DNA
   <213> Artificial Sequence
<220>
   <223> Description of Artificial Sequence: 3'-primer
<400> 14
   ttttggatcc ggactcggac tcaaacatct tcttccg 37
<210> 15
   <211> 37
   <212> DNA
   <213> Artificial Sequence
<220>
   <223> Description of Artificial Sequence: 5'-primer
<400> 15
   ttttggatcc ggagatgagg agaagcggaa cagggcc 37
<210> 16
   <211> 37
   <212> DNA
   <213> Artificial Sequence
<220>
   <223> Description of Artificial Sequence: 3'-primer
<400> 16
   ttttaagctt ctaggactcg gactcaaaca tcttctt 37
<210> 17
   <211> 37
   <212> DNA
   <213> Artificial Sequence
<220>
   <223> Description of Artificial Sequence: 5'-primer
<400> 17
   ttttggatcc aagtgtatat tgttaaagtg gatactg 37
<210> 18
   <211> 37
   <212> DNA
   <213> Artificial Sequence
<220>
   <223> Description of Artificial Sequence: 3'-primer
<400> 18
   ttttaagctt tcattcacca gttgtatata aagactg 37